Europäisches Patentamt

European Patent Office

Office européen des brevets

(11) Veröffentlichungsnummer: **0 164 374**
**B1**

(12) **EUROPÄISCHE PATENTSCHRIFT**

(45) Veröffentlichungstag der Patentschrift:
**28.10.87**

(51) Int. Cl.⁴: **A 61 F 5/01**

(21) Anmeldenummer: **85900056.4**

(22) Anmeldetag: **06.12.84**

(86) Internationale Anmeldenummer:
**PCT/DE 84/00264**

(87) Internationale Veröffentlichungsnummer:
**WO 85/02537 (20.06.85 Gazette 85/14)**

(54) **ORTHESE FÜR KNIEGELENKE.**

(30) Priorität: **08.12.83 DE 3344422**

(43) Veröffentlichungstag der Anmeldung:
**18.12.85 Patentblatt 85/51**

(45) Bekanntmachung des Hinweises auf die Patenterteilung:
**28.10.87 Patentblatt 87/44**

(84) Benannte Vertragsstaaten:
**BE CH DE FR GB LI NL SE**

(56) Entgegenhaltungen:
**DE - C - 544 634**
**US - A - 2 195 024**
**US - A - 3 046 981**

(73) Patentinhaber: **S + G IMPLANTS GMBH,**
**Grapengiesserstrasse 21, D-2400 Lübeck (DE)**

(72) Erfinder: **HENSSGE, Ernst-Joachim, Im Trentsaal 7,**
**D-2400 Lübeck (DE)**
Erfinder: **GRUNDEI, Hans, Gärtnergasse 4,**
**D-2400 Lübeck (DE)**

(74) Vertreter: **Wilcken, Thomas, Dipl.-Ing. et al,**
**Musterbahn 1, D-2400 Lübeck (DE)**

## Beschreibung

Die Erfindung betrifft eine Orthese zum Schienen und zur Kompensation der seitlichen Instabilität von Kniegelenken, bei der eine an der Kniegelenkseite mit der überwiegenden Instabilität anzulegende Pelotte vorgesehen ist, an der elastische Gurtbänder angreifen, die zu oberhalb und unterhalb sowie seitlich zu der Pelotte versetzt befindlichen Befestigungsstellen führen, wobei sich die Wirkungslinien jeweils zweier, einander benachbarter Gurtbänder im Bereich der Pelotte kreuzen.

In der DE-C-544 634 ist eine Orthese der vorgenannten Art beschrieben. Des weiteren sind Orthesen bekannt, die aus einem oberen und aus einem unteren, den menschlichen Ober- bzw. Unterschenkel mehr oder weniger umfassenden Schienenteil aus starrem Material bestehen, wobei die beiden Schienenteile durch ein scharnierartiges Gelenk miteinander verbunden sind.

Nachteilig bei diesen bekannten Orthesen ist, dass sie den natürlichen, kombinierten Abroll- und Gleitvorgang des physiologischen Bewegungsablaufes beim Beugen und Strecken des menschlichen Knies nicht gestatten, was zu einer unnatürlichen und schädlichen Belastung der gelenkbildenden Knochenfläche des Ober- und Unterschenkelknochens führt und sich am Ober- und Unterschenkel eine muskuläre Atrophie einstellt. Dies ist eine Folge der seitlichen Unnachgiebigkeit der Orthese. Weiterhin führt die dem Knie durch diese vorbekannten Orthesen aufgezwungene Kniebewegung, insbesondere auch wegen der seitlichen Unnachgiebigkeit der Orthesen, zu dem Nachteil, dass die Orthesen vom Patienten im Tragen unangenehm empfunden werden.

Die Aufgabe der Erfindung besteht in der Schaffung einer Orthese der einleitend angeführten Art, die den natürlichen Bewegungsablauf des Knies gestattet und angenehm im Tragen ist.

Die Lösung dieser Aufgabe geht von der einleitend angeführten Orthese aus und kennzeichnet sich dadurch, dass die Befestigungsstellen für Gurtbänder an zwei Schellen vorgesehen sind, von denen bei angelegter Orthese die eine Schelle den Oberschenkel und die andere Schelle den Unterschenkel des Patienten teilweise umfasst und deren offener Bereich mit lösbaren Bändern überbrückbar ist, und dass die Pelotte und die beiden Schellen durch mindestens einen sie verbindenden, biegeelastischen Stab auf Abstand gehalten sind.

Eine bevorzugte Ausgestaltung der erfindungsgemässen Orthese besteht darin, dass an der Pelotte Ösen schwenkbar angebracht sind, durch welche die an der Pelotte angreifenden Gurtbänder gesteckt sind, und dass der Abstand der beiden Schellen und der Pelotte zueinander variabel ist.

Durch diese Lösung ist der natürliche Bewegungsablauf der gelenkbildenden Knochenflächen von Ober- und Unterschenkelknochen bei guter Kompensation der lateralen Instabilität des Kniegelenkes gewährleistet, da dem Kniegelenk durch die Orthese keinerlei Bewegung aufgezwungen wird. Somit wird die erwähnte Muskelatrophie vermieden. Weiterhin ist die erfindungsgemässe Orthese angenehm zu tragen, da die Pelotte und die an ihr angreifenden Bänder den individuellen Bewegungen aller im Bereich des Knies an dessen Bewegung beteiligten Knieteile nachgehen können, ohne dass unangenehme Druckempfindungen oder dergleichen entstehen. Dadurch wird auch beim Patienten die Bereitschaft gefördert, die erfindungsgemässe Orthese bereitwillig zu tragen, die erfindungsgemässe Orthese bereitwillig zu tragen, was wiederum der medizinischen Absicht zugute kommt.

Die Erfindung ist nachstehend anhand eines in den anliegenden Zeichnungen dargestellten Ausführungsbeispieles näher erläutert. Es zeigen:

Fig. 1 das Ausführungsbeispiel in einer ersten Seitenansicht,

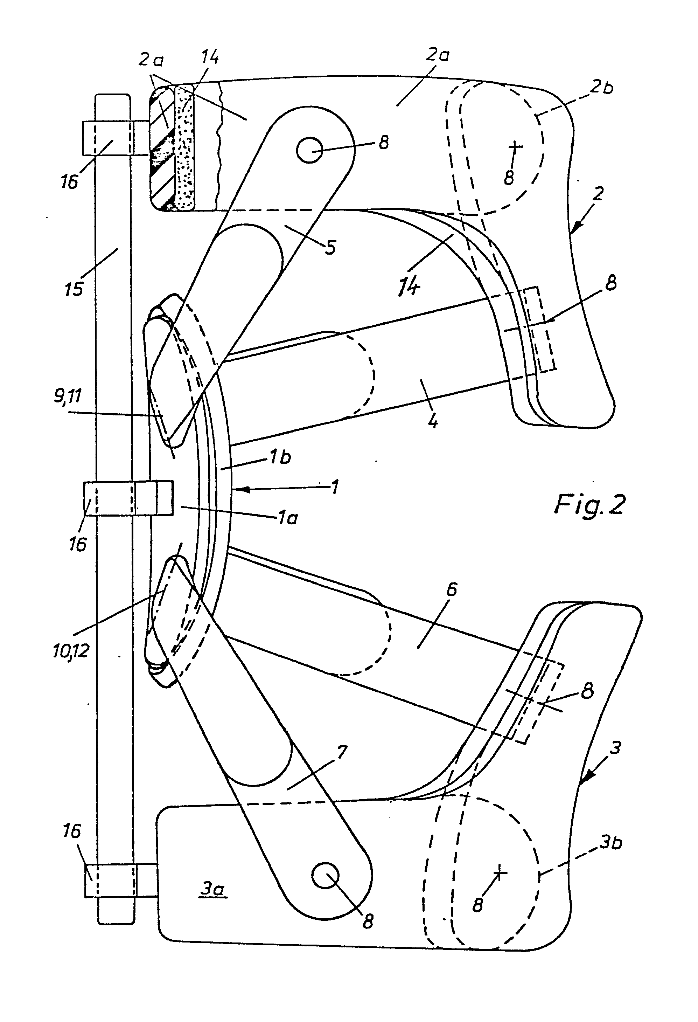

Fig. 2 eine weitere Seitenansicht des Ausführungsbeispiels gemäss dem Pfeil A in Fig. 1.

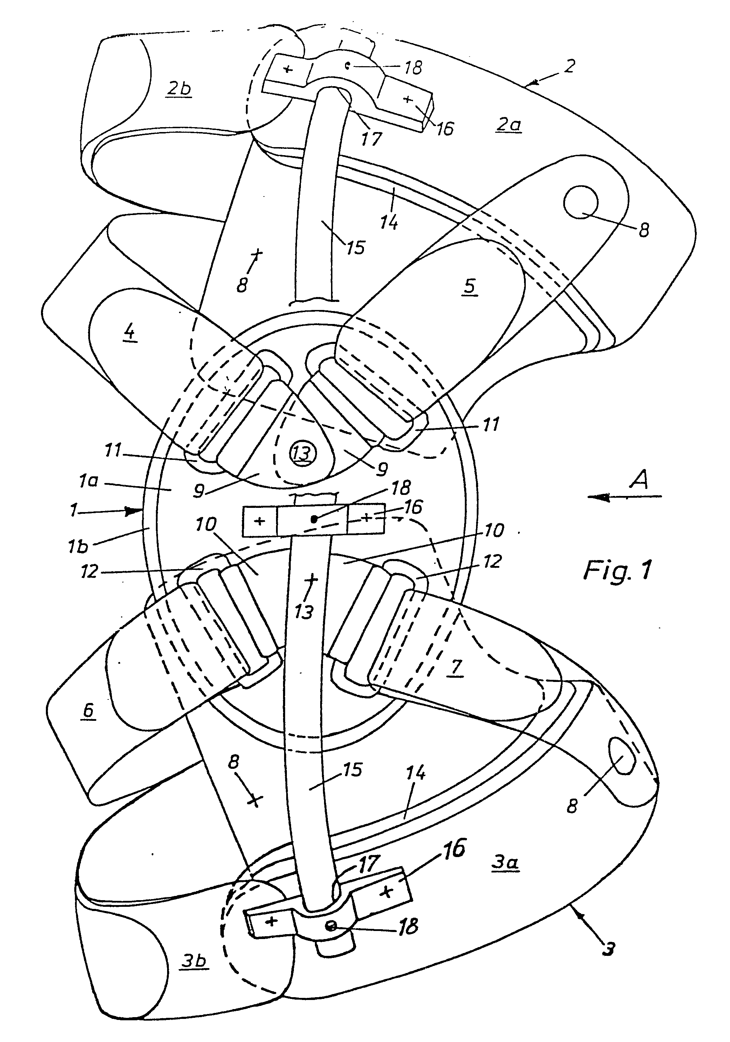

Gemäss den Figuren besteht die dargestellte, z.B. nach Kniebandplastiken vom Patienten im Bereich des Knies zu tragende Orthese aus einer Pelotte 1, einer oberen Schelle 2, einer unteren Schelle 3 und aus mehreren elastischen Gurtbändern 4, 5, 6 und 7. Die Gurtbänder greifen einerseits an der Pelotte 1 an und sind andererseits an Befestigungsstellen der beiden Schellen 2, 3 befestigt, z.B. durch Nieten 8, wobei sich diese Stellen in bezug auf die Pelotte seitlich versetzt an den Schellen befinden, wie Fig. 1 deutlich zeigt. Die Festlegung der Gurtbänder an der Pelotte ist lösbar und kann z.B. derart erfolgen, dass Laschen 9, 10 mit Ösen 11, 12 versehen sind, wobei die Gurtbänder wie gezeichnet durch die Ösen hindurchgesteckt sind. Jeweils zwei der Laschen 9 und 10 sind z.B. mittels einer Niet 13 an der Pelotte 1 befestigt. Des weiteren verlaufen die Gurtbänder von den Schellen 2, 3 so in Richtung zu der Pelotte, dass sich die Wirkungslinien jeweils zweier, einander benachbarter Gurtbänder im Bereich der Pelotte kreuzen, wie aus Fig. 1 klar ersichtlich ist: Hierbei sei noch erwähnt, dass die Gurtbänder bzw. Laschen durch die Nieten 8 bzw. 13 so befestigt sind, dass eine Verschwenkung dieser Teile um die Nietachse möglich ist, damit die Gurtbänder beim Anlegen und Tragen der Orthese in einem gewissen Mass beweglich sind.

Die sowohl längs- als auch querelastischen Gurtbänder 4, 5, 6 und 7 sind ferner so ausgebildet, dass sie auf einer Seite mit einer sogenannten Klettverschlussausbildung versehen sind. Aufbau und Funktion einer solchen Verschlussausbildung sind allgemein bekannt, so dass sich eine genaue Beschreibung hierüber erübrigt.

Die beiden Schellen 2 und 3 bestehen je aus einem relativ steifen Materialteil 2a bzw. 3a, z.B. aus Kunststoff, und aus einem flexiblem Verschlussband 2b bzw. 3b, wobei das eine Ende des Bandes an dem Materialteil z.B. durch Nietung befestigt ist, während dessen anderes Ende an dem Materialteil lösbar befestigt ist. Letzteres kann wiederum in der Art und Weise erfolgen, wie es in Verbindung mit den Gurtbändern 4 bis 7 beschrieben und gezeichnet ist. Lediglich aus Überschichtlichkeitsgründen ist dies in den Zeichnungen nicht dargestellt. Wenn die Orthese am Bein eines Patienten angelegt ist, umfassen die in axialer Richtung steifen, jedoch in radialer Richtung flexiblen Materialteile 2a, 3a, der Schellen 2 bzw. 3 den Oberschenkel bzw. Unterschenkel des Patienten jeweils teilweise, während die Verschlussbänder 2b, 3b, die ebenfalls mit einer Klettverschlussausbildung versehen sind, die Schenkel im übrigen überbrücken.

Die Pelotte 1 besteht aus einem starren Materialteil 1a aus z.B. Kunststoff und aus einem nachgiebigen Polsterbelag 1b aus z.B. einem Schaumstoff, der auf der dem Knie des Patienten zugewandten Seite des starren Materialteils vorzugsweise durch Kleben befestigt ist.

Auch die starren Materialteile 2a, 3a der Schellen 2 und 3 sind innenseitig mit einem nachgiebigen Polsterbelag 14 aus z.B. Schaumstoff versehen, um eine angenehme Anlage der Schellen am Bein des Patienten zu erreichen.

Um die Schellen 2, 3 relativ zu der Pelotte 1 auf Abstand zu halten, ist wenigstens ein biegeelastischer Stab 15 aus vorzugsweise Kunststoff vorgesehen, der mit Hilfe von angenieteten Befestigungselementen 16 an den Schellen und an der Pelotte angebracht ist. Die z.B. in Form eines Augenlagers aufgebauten Elemente 16 weisen ein Loch 17 auf, durch das der Stab 15 hindurchgesteckt ist. Um den Stab in den Elementen 16 axial zu sichern, weisen letztere Gewindelöcher auf, in die von aussen betätigbare Schrauben 18 eingreifen, die gegen den Stab geklemmt werden. Durch diese Festlegeart des Stabes in den Elementen kann der Abstand zwischen den Schellen und der Pelotte entsprechend den Verhältnissen am Knie des Patienten eingestellt werden. Die Querschnittsform des Stabes 15 kann kreisförmig, aber auch oval sein. letzteres wird angewendet werden, wenn ein stärkerer Biegewiderstand des Stabes wegen erhöhter Instabilität erwünscht ist, wobei die grosse Achse eines ovalen Stabes bei angelegter Orthese zur Kniegelenkachse des Patienten im wesentlichen parallel verläuft.

## Patentansprüche

1. Orthese zum Schienen und zur Kompensation der seitlichen Instabilität von Kniegelenken, bei der eine an der Kniegelenkseite mit der überwiegenden Instabilität anzulegende Pelotte (1) vorgesehen ist, an der elastische Gurtbänder (4, 5, 6, 7) angreifen, die zu oberhalb und unterhalb sowie seitlich zu der Pelotte (1) versetzt befindlichen Befestigungsstellen (8) führen, wobei die Wirkungslinien jeweils zweier, einander benachbarter Gurtbänder im Bereich der Pelotte kreuzen, dadurch gekennzeichnet, dass die Befestigungsstellen (8) für die Gurtbänder an zwei Schellen (2; 3) vorgesehen sind, von denen bei angelegter Orthese die eine Schelle (2) den Oberschenkel und die andere Schelle (3) den Unterschenkel des Patienten teilweise umfasst und deren offener Bereich mit lösbaren Bändern (2b; 3b) überbrückbar ist, und dass die Pelotte (1) und die beiden Schellen (2; 3) durch mindestens einen sie verbindenden, biegeelastischen Stab (15) auf Abstand gehalten sind.

2. Orthese nach Anspruch 1, dadurch gekennzeichnet, dass an der Pelotte (1) Ösen (11, 12) schwenkbar angebracht sind, durch welche die an der Pelotte angreifenden Gurtbänder (4, 5, 6, 7) gesteckt sind.

3. Orthese nach Anspruch 1 und 2, dadurch gekennzeichnet, dass die Gurtbänder (4, 5, 6, 7) unlösbar an den Schellen (2; 3) und lösbare an der Pelotte (1) befestigt sind.

4. Orthese nach einem der Ansprüche 1 bis 3, dadurch gekennzeichnet, dass der Abstand der beiden Schellen (2; 3) und der Pelotte (1) zueinander variabel ist.

5. Orthese nach Anspruch 1 oder 4, dadurch gekennzeichnet, dass der Stab (15) einen ovalen Querschnitt aufweist.

6. Orthese nach Anspruch 1, 4 oder 5, dadurch gekennzeichnet, dass der Stab (15) durch Löcher (17) von Befestigungselementen (16) verläuft, die an der Pelotte (1) und den Schellen (2; 3) befestigt sind und in denen von aussen betätigbare Schrauben (18) sitzen, mit denen der Stab im jeweiligen Befestigungselement festklemmbar ist.

## Claims

1. Brace for splinting and compensation of the lateral instability of knee joints, in which is provided a pad (1) to be positioned on the knee joint side having the preponderant instability, which is acted upon by elastic belt straps (4, 5, 6, 7) which lead to fastening points (8) situated above and below as well as laterally offset with respect to the pad (1), whereby the lines of action of every two belt straps adjacent to one another intersect in the region of the pad, characterised in that the fastening points (8) for the belt straps are provided on two collars (2; 3) of which the one collar (2) partially surrounds the upper segment of the leg and the other collar (3) partially surrounds the lower segment of the leg of the patient when the brace is applied, and the open portions of which are bridgeable by means or releasable straps (2b; 3b) and that the pad (1) and the two (2; 3) are held spaced apart by means of at least one flexurally elastic bar (15) connecting them.

2. Brace according to claim 1, characterised in that lugs (11, 12) through which the belt straps (4, 5, 6, 7) acting on the pads are passed, are pivotally attached to the pad (1).

3. Brace according to claims 1 and 2, characterised in that the belt straps (4, 5, 6, 7) are non-releasably attached to the collars (2; 3) and releasably to the pad (1).

4. Brace according to one of the claims 1 to 3, characterised in that the spacing of the two collars (2; 3) and of the pad (1) from one another is variable.

5. Brace according to claim 1 or 4, characterised in that the bar (15) has an oval cross-section.

6. Brace according to claim 1, 4 or 5, characterised in that the bar (15) extends through holes (17) of fastening elements (16) which are secured to the pad (1) and the collars (2; 3) and wherein are situated screws (18) operable from the outside, by means of which the bar may be clamped fast in the respective fastening element.

## Revendications

1. Prothèse orthopédique destinée à avoir un effet d'attelle et compenser l'instabilité latérale de l'articulation du genou, et dans laquelle il est prévu une calotte (1), qui s'applique sur le côté de l'articulation du genou, sur lequel l'instabilité est prépondérante, et sur laquelle s'accrochent des sangles élastiques (4, 5, 6, 7), qui aboutissent à des points de fixation (8) disposés en étant décalés au-dessus, au-dessous et latéralement par rapport à la calotte (1), les lignes d'action de deux sangles respectives, qui sont voisines, se croisant dans la zone de la calotte, caractérisée en ce que les points de fixation (8) pour les sangles sont prévus sur deux coques (2; 3) qui, lorsque la prothèse est mise en place, entourent partiellement, l'une (2), la cuisse et l'autre (3), la partie inférieure de la jambe du patient, et dont la zone que ces coques laissent libre, peut être enserrée par des bandes détachables (2b; 3b), et que la calotte (1) et les deux coques (2, 3) sont maintenues à distance par au moins une barre flexible (15), qui les relie.

2. Prothèse orthopédique selon la revendication 1, caractérisée en ce que sur la calotte (1) se trouvent installés des oeillets (11, 12) qui peuvent pivoter et dans lesquels se trouvent enfichées les angles (4, 5, 6, 7) qui sont accrochées à la calotte.

3. Prothèse orthopédique selon les revendications 1 et 2, caractérisée en ce que les sangles (4, 5, 6, 7) sont fixées d'une manière non détachable aux coques (2; 3) et de façon détachable à la calotte (1).

4. Prothèse orthopédique selon l'une des revendications 1 à 3, caractérisée en ce que la distance entre les deux coques (2, 3) et la calotte (1) est variable.

5. Prothèse orthopédique selon la revendication 1 ou 4, caractérisée en ce que le barreau (15) possède une section transversale ovale.

6. Prothèse orthopédique selon la revendication 1, 4 ou 5, caractérisée en ce que le barreau (15) s'étend à travers des trous (17) ménagés dans des organes de fixation (16) qui sont fixés sur la calotte (1) et sur les coques (2; 3) et dans lesquels s'engagent des vis (18) pouvant être manœuvrées de l'extérieur et à l'aide desquelles la barre peut être bloquée par serrage dans l'élément de fixation respectif.

0 164 374

Fig. 1

5

Fig.2